Europäisches Patentamt

European Patent Office

Office européen des brevets

(11) Veröffentlichungsnummer: **0 267 501 B1**

# (12) EUROPÄISCHE PATENTSCHRIFT

(45) Veröffentlichungstag der Patentschrift: **16.10.91**

(51) Int. Cl.⁵: **C07C 67/38, B01J 31/20**

(21) Anmeldenummer: **87115961.2**

(22) Anmeldetag: **30.10.87**

(54) **Verfahren zur kontinuierlichen Herstellung von Carbalkoxygruppen enthaltenden aliphatischen Verbindungen.**

(30) Priorität: **08.11.86 DE 3638219**

(43) Veröffentlichungstag der Anmeldung:
**18.05.88 Patentblatt 88/20**

(45) Bekanntmachung des Hinweises auf die
Patenterteilung:
**16.10.91 Patentblatt 91/42**

(84) Benannte Vertragsstaaten:
**BE CH DE ES FR GB IT LI NL**

(56) Entgegenhaltungen:
**EP-A- 0 042 928**
**DE-A- 2 948 888**

(73) Patentinhaber: **BASF Aktiengesellschaft**
**Carl-Bosch-Strasse 38**
**W-6700 Ludwigshafen(DE)**

(72) Erfinder: **Maerkl, Robert, Dr.**
**Schulstrasse 17**
**W-6701 Fussgoenheim(DE)**
Erfinder: **Bertleff, Werner, Dr.**
**Neuzenlache 3**
**W-6806 Viernheim(DE)**
Erfinder: **Kühn, Gebhard**
**Am Weidenschlag 92**
**W-6700 Ludwigshafen(DE)**
Erfinder: **Panitz, Paul**
**Wachenheimer Strasse 1**
**W-6520 Worms 1(DE)**
Erfinder: **Stops, Peter**
**Limburgstrasse 12**
**W-6701 Altrip(DE)**
Erfinder: **Kummer, Rudolf, Dr.**
**Kreuzstrasse 6**
**W-6710 Frankenthal(DE)**
Erfinder: **Schuch, Günter, Dr.**
**Ullrich-von-Hutten-Strasse 5**
**W-6700 Ludwigshafen(DE)**

## Beschreibung

Gegenstand der Erfindung ist ein Verfahren zur kontinuierlichen Herstellung von Carbalkoxygruppen enthaltenden aliphatischen Verbindungen durch Umsetzen von olefinisch ungesättigten aliphatischen Verbindungen mit Kohlenmonoxid und Alkanolen in Gegenwart von Cobaltcarbonylkatalysatoren und tert. Stickstoffbasen.

Aus der DE-OS 16 18 156 ist ein Verfahren bekannt, bei dem man Olefine oder Alkencarbonsäureester durch Umsetzen mit Kohlenmonoxid und Alkanolen in Gegenwart von Cobaltcarbonylkatalysatoren und heterocyclischen aromatischen tert. Stickstoffbasen carbalkoxyliert. Bei der technischen Durchführung des Verfahrens wird das im Überschuß angewandte Kohlenmonoxid, zweckmäßig unter Reaktionsdruck, wieder verwendet. Hierbei kommt es zu Abscheidungen von Cobaltoxalat und zu einer Verminderung der Reaktionsgeschwindigkeit bei der Carbalkoxylierungsreaktion.

Es war deshalb die technische Aufgabe gestellt, ein Verfahren zur Herstellung von Carbalkoxygruppen enthaltenden aliphatischen Verbindungen durch Carbalkoxylierung von olefinisch ungesättigten aliphatischen Verbindungen zur Verfügung zu stellen, bei dem die Abscheidung von Cobaltoxalat vermieden und die Reaktionsgeschwindigkeit möglichst nicht beeinträchtigt wird.

Diese technische Aufgabe wird gelöst in einem Verfahren zur Herstellung von Carbalkoxygruppen enthaltenden aliphatischen Verbindungen durch Umsetzung von olefinisch ungesättigten aliphatischen Verbindungen mit Kohlenmonoxid und Alkanolen in Gegenwart von Cobaltcarbonylkatalysatoren und tert. Stickstoffbasen bei einer Temperatur von 80 bis 200°C und unter einem Druck von 100 bis 1200 bar, wobei man Kohlenmonoxid im Kreis führt und im Kohlenmonoxid, das der Reaktion zugeführt wird, einen Gehalt an Kohlendioxid von 0,1 bis 2,0 Vol.% einhält.

Das neue Verfahren hat den Vorteil, daß die Reaktionsgeschwindigkeit bei Carbalkoxylierung nicht beeinträchtigt wird, zudem die Abscheidungen von Cobaltoxalat vermieden werden.

In der Regel haben die als Ausgangsstoffe verwendeten olefinisch ungesättigten Verbindungen, abgesehen von Carbalkoxygruppen als Substituenten, Kohlenwasserstoffstruktur. Bevorzugte Ausgangsstoffe sind Olefine und Diolefine mit 4 bis 16 Kohlenstoffatomen, die auch eine Carbalkoxygruppe mit 2 bis 5 Kohlenstoffatomen als Substituenten haben können, insbesondere alpha-Olefine mit 4 bis 10 Kohlenstoffatomen oder Ester von Alkencarbonsäuren mit 4 bis 11 Kohlenstoffatomen. Geeignete Ausgangsstoffe sind beispielsweise Isobutylen, Buten, 1-Buten, 2-Octen, 1-Octen, -Diisobutylen, Hexen-1, Butadien, Hexadien, Undecylensäureester oder Pent-3-ensäuremethylester, insbesondere Butadien und Butadien enthaltende $C_4$-Schnitte, z.B. mit einem Gehalt an Butadien von 30 bis 70 Gew.-%.

Bevorzugte Erzeugnisse sind demzufolge Alkancarbonsäureester, Alkencarbonsäureester sowie Alkandicarbonsäureester.

Geeignete Alkanole haben vorteilhaft 1 bis 6 Kohlenstoffatome, insbesondere 1 bis 4 Kohlenstoffatome. Beispielhaft seien genannt Methanol, Ethanol, Isopropanol, Butanol oder Hexanol. Besonders bevorzugt ist Methanol.

Die Alkanole wendet man in der Regel in Überschuß an. Vorteilhaft wendet man je Mol olefinisch ungesättigte Verbindung 1,5 bis 10 Mol, insbesondere 2 bis 5 Mol Alkanole an.

Die Umsetzung wird bei einer Temperatur von 80 bis 200°C durchgeführt. Besonders gute Ergebnisse erhält man bei einer Temperatur von 100 bis 180°C. Ferner hält man bei der Umsetzung einen Druck von 100 bis 1200 bar, insbesondere von 150 bis 900 bar ein.

Kohlenmonoxid wird vorteilhaft im Überschuß, z.B. dem 1,5- bis 10-fachen der stöchiometrisch erforderlichen Menge angewandt. Kohlenmonoxid wird fortwährend im Kreis geführt. Erfindungsgemäß beträgt im Kohlenmonoxid, das der Reaktion zugeführt wird (Gemisch aus Kreisgas und frischem Kohlenmonoxid), der Gehalt an Kohlendioxid von 0,1 bis 2,0 Vol.%, insbesondere von 0,5 bis 1,8 Vol.%.

Die verwendeten Cobaltcarbonylkatalysatoren werden entweder in situ aus Cobaltsalzen, z.B. fettsauren Cobaltsalzen, wie Formiat, Acetat, Propionat oder Butyrat erzeugt. Vorteilhaft bringt man den Katalysator bereits als Cobaltcarbonyl ein. Insbesondere hat es sich bewährt, wenn man Cobaltcarbonylkatalysator gelöst in den olefinisch ungesättigten Ausgangsverbindungen in das Reaktionsgemisch einbringt. Eine solche Lösung erhält man beispielsweise durch Umsetzen einer wäßrigen Lösung von fettsauren Cobaltsalzen mit einem Gemisch aus Kohlemonoxid und Wasserstoff in Gegenwart von Aktivkohle bei einer Temperatur von 100 bis 170°C und unter einem Druck von 100 bis 400 bar. Aus der wäßrigen Lösung wird das entstandene Cobaltcarbonyl dann mit der olefinisch ungesättigten Verbindung extrahiert.

Die Umsetzung wird in Gegenwart tert. Stickstoffbasen, insbesondere von heterocyclischen aromatischen tertiären Stickstoffbasen vorteilhaft mit einem $pk_a$-Wert von 6 bis 9 durchgeführt.

Geeignete Stickstoffbasen sind beispielsweise 3-Methylpyridin ($pk_a$ 6,0) 4-Methylpyridin ($pk_a$ 6,0), 2,3-Dimethylpyridin ($pk_a$ 6,6) 2,4-Dimethyl-pyridin ($pk_a$ 7,0) oder 3,5-Dimethylpyridin ($pk_a$ 6,2). Besondere technische Bedeutungen haben 3-Methylpyridin und 4-Methylpyridin oder deren Gemische erlangt. Besonders bewährt hat es sich, wenn man je Mol Cobaltcarbonylkatalysator 2 bis 25 Mol der vorgenannten Stickstoffbasen anwendet.

Das Verfahren nach der Erfindung führt man beispielsweise aus, indem man in mindestens einem Hochdruckgefäß aus nichtrostendem Stahl, in dem für gute Vermischung gesorgt wird, z.B. zwei hintereinander geschalteten Schlaufenreaktoren, fortlaufend olefinisch ungesättigte Verbindungen, Alkanole, Cobaltcarbonylkatalysatoren, heterocyclische aromatische tertiäre Stickstoffbasen sowie Kchlenmonoxid in den angegebenen Verhältnissen zuführt und das Reaktionsgemisch auf die angegebene Temperatur und den angegebenen Druck hält. In dem Maße wie Ausgangsstoffe zugeführt werden, wird Reaktionsgemisch entnommen und Kohlenmonoxid abgetrennt und wiederum mit frischem Kohlenmonoxid dem Ausgangsgemisch zugesetzt. Das verbleibende Reaktionsgemisch wird nach Entspannen durch Abtrennen des Cobaltkatalysators, z.B. durch Behandeln in wäßrigsaurem Medium mit molekularem Sauerstoff enthaltenden Gasen, Abtrennen der Kobaltsalzlösung und Destillation der organischen Phase aufgearbeitet. Die nach dem Verfahren der Erfindung hergestellten Carbonsäureester eignen sich als Ausgangsstoffe für Faserrohstoffe oder als Weichmacher für Hochpolymere.

Das Verfahren nach der Erfindung sei in folgenden Beispielen veranschaulicht. Die angegebenen Gewichtsteile verhalten sich zu den Volumenteilen wie Kilogramm zu Liter.

Beispiel 1

a) In ein Hochdruckgefäß von 100 Volumenteilen führt man von unten stündlich 33 Gewichtsteile $C_4$-Schnitt mit 40 % (m/m) Butadien-1,3, 27 Gewichtsteile 3-Methylpyridin, 11 Gewichtsteile Methanol, 1 Gewichtsteil Cobalt in Form von Kobaltcarbonylverbindungen und 28 Gewichtsteile eines Gasgemisches mit folgender Zusammensetzung:

83,5 % Kohlenmonoxid
1,3 % Kohlendioxid
2,6 % Stickstoff
12,5 % Butene zu.

Die Carbonylierung verläuft bei einer Temperatur von 135 °C und 650 bar. Das am Kopf des Hochdruckgefässes abgenommene Produkt wird entspannt und das abgetrennte Kreisgas in die Synthese zurückgeführt. Anschließend werden die überschüssigen $C_4$-Kohlenwasserstoffe abgetrennt. Sie enthalten 1000 ppm Butadien, was einem Umsatz von 99,9 % entspricht.

b) Unter vermindertem Druck, um den Katalysator zu schonen, werden vom Austrag Methanol, 3-Pentensäuremethylester und der größte Teil des 3-Methylpyridins abdestilliert.

c) Der so erhaltene Destillationssumpf wird mit 3-Pentensäuremethylester und Methanol verdünnt. Dieser Reaktionslösung, die 55 Gewichtsteile 3-Pentensäuremethylester, 46 Gewichtsteile 3-Methylpyridin, 23 Gewichtsteile Methanol, 1 Gewichtsteil Cobalt in Form von Kobaltcarbonylverbindungen enthält, werden 16 Gewichtsteile Kohlenmonoxid zugesetzt und das Gemisch einem weiteren Hochdruckgefäß kontinuierlich von unten zugeführt. Die Carbonylierung wird bei einer Temperatur von 165 °C und einem Druck von 150 bar durchgeführt. Der Umsatz des 3-Pentensäuremethylesters beträgt 72 %, die Selektivität zu Adipinsäuredimethylester 79 %. Das am Kopf des Hochdruckgefäßes abgenommene Produkt wird entspannt, wobei überschüssiges Kohlenmonoxid gasförmig entweicht. Es enthält 5 % (m/m) Kohlendioxid. Dieser Gasstrom wird in die 1. Carbonylierungsstufe zurückgeführt und mit frischem Kohlenmonoxid gemischt, so daß der Kohlendioxidgehalt des Gemisches, das der ersten Reaktionsstufe zugeführt wird, 1,3 Vol.% beträgt.

Beispiel 2 (Vergleichsbeispiel)

Entsprechend dem Beispiel 1 wird Adipinsäuredimethylester zweistufig aus Butadien, Kohlenmonoxid und Methanol kontinuierlich hergestellt.

Im Abgas der Stufe 1c) sind jedoch 15 % (m/m) Kohlendioxid enthalten. Dies führt bei unveränderter Menge an frischem Kohlenmonoxid zu einem Kohlendioxidgehalt von 2,7 % (m/m) im Gasgemisch, das der Stufe 1a) zugeführt wird. Der Umsatz in der Stufe 1a) erniedrigt sich auf 99,6 %. Bei der Innenbesichtigung der Hochdruckgefäße wurde ein Niederschlag festgestellt, der entsprechend der Analyse als Cobaltoxalat identifiziert wurde.

**Patentansprüche**

1.  Verfahren zur kontinuierlichen Herstellung von Carbalkoxygruppen enthaltenden aliphatischen Verbindungen durch Umsetzen von olefinisch ungesättigten aliphatischen Verbindungen mit Kohlenmonoxid und Alkanolen in Gegenwart von Cobaltcarbonylkatalysatoren und tertiären Stickstoffbasen bei einer Temperatur von 80 bis 200 °C und unter einem Druck von 100 bis 1200 bar, dadurch gekennzeichnet, daß man Kohlenmonoxid im Kreis führt und im Kohlenmonoxid, das der Reaktion zugeführt wird, einen Gehalt an Kohlendioxid von 0.1 bis 2.0 Vol.-% einhält.

**2.** Verfahren nach Anspruch 1, dadurch gekennzeichnet, daß man heterocyclische aromatische tertiäre Stickstoffbasen mit einem $pk_a$-Wert von 6 bis 9 verwendet.

**3.** Verfahren nach den Ansprüchen 1 und 2, dadurch gekennzeichnet, daß man alpha-Olefine mit 4 bis 10 Kohlenstoffatomen als Ausgangsstoffe verwendet.

**4.** Verfahren nach den Ansprüchen 1 und 2, dadurch gekennzeichnet, daß man Butadien oder Butadien enthaltenden C$_4$-Schnitt verwendet.

**5.** Verfahren nach den Ansprüchen 1 und 2, dadurch gekennzeichnet, daß man Ester von Alkencarbonsäuren mit 4 bis 11 Kohlenstoffatomen als Ausgangsstoffe verwendet.

**6.** Verfahren nach den Ansprüchen 1 bis 5, dadurch gekennzeichnet, daß man 3-Methylpyridin, 4-Methylpyridin oder deren Gemische mitverwendet.

## Claims

**1.** A process for the continuous preparation of a carbalkoxy-containing aliphatic compound by reacting an olefinically unsaturated aliphatic compound with carbon monoxide and an alkanol in the presence of a cobalt carbonyl catalyst and a tertiary nitrogen base at from 80 to 200°C and under from 100 to 1,200 bar, wherein carbon monoxide is circulated, and a carbon dioxide content of from 0.1 to 2.0% by volume is maintained in the carbon monoxide fed to the reaction.

**2.** A process as claimed in claim 1, wherein the heterocyclic aromatic tertiary nitrogen base having a $pK_a$ of from 6 to 9 is used.

**3.** A process as claimed in claims 1 and 2, wherein an alpha-olefin of 4 to 10 carbon atoms is used as a starting material.

**4.** A process as claimed in claims 1 and 2, wherein butadiene or a butadiene-containing C$_4$ cut is used.

**5.** A process as claimed in claims 1 and 2, wherein an ester of an alkenecarboxylic acid of 4 to 11 carbon atoms is used as a starting material.

**6.** A process as claimed in any of claims 1 to 5, wherein 3-methylpyridine, 4-methylpyridine or a mixture thereof is present.

## Revendications

**1.** Procédé de préparation continue de composés aliphatiques contenant des groupements carbalcoxy, par réaction de composés aliphatiques insaturés oléfiniquement avec du monoxyde de carbone et des alcanols en présence de catalyseurs à base de cobaltcarbonyle et de bases azotées tertiaires, à une température de 80 à 200°C et sous une pression de 100 à 1200 bar, caractérisé en ce qu'on fait circuler du monoxyde de carbone et en ce qu'on maintient, dans le monoxyde de carbone qui est envoyé dans la réaction, une teneur en anhydride carbonique de 0,1 à 2,0% en volume.

**2.** Procédé selon la revendication 1, caractérisé en ce qu'on utilise des bases azotées tertiaires aromatiques hétérocycliques ayant un $pK_a$ de 6 à 9.

**3.** Procédé selon la revendication 1 ou 2, caractérisé en ce qu'on utilise, comme substances de départ, des $\alpha$-oléfines à 4-10 atomes de carbone.

**4.** Procédé selon la revendication 1 ou 2, caractérisé en ce qu'on utilise du butadiène ou une coupe en C$_4$ contenant du butadiène.

**5.** Procédé selon la revendication 1 ou 2, caractérisé en ce qu'on utilise, comme substances de départ, des esters d'acides alcènecarboxyliques à 4-11 atomes de carbone.

**6.** Procédé selon l'une quelconque des revendications 1 à 5, caractérisé en ce qu'on utilise simultanément de la 3-méthylpyridine, de la 4-méthylpyridine ou des mélanges de celles-ci.